# EUROPEAN PATENT APPLICATION

(11) **EP 2 090 172 A1**
(43) Date of publication of application: **19.08.2009**
(21) Application number: 07254262.4
(22) Date of filing: 29.10.2007
(51) Int. Cl.: A21D 2/14, A21D 2/16, A23L 1/30, A61K 31/202, A61P 3/04

(54) **Dough composition**

(71) Applicant: Lipid Nutrition B.V., 1521 AZ Wormerveer (NL)
(72) Inventor: Schmid, Ulrike, 1521 AZ Wormerveer (NL); Mulder, Ellen Maria Elizabeth, 1521 AZ Wormerveer (NL); Van Wanroij, Miriam Aldegonda Josephina, 1521 AZ Wormerveer (NL)
(74) Representative: Probert, Gareth David

(57) **Abstract**

A dough composition comprising flour and a fat phase, wherein the fat phase. comprises from 5 to 35% by weight pinolenic acid or a derivative thereof. There is also disclosed a dough composition comprising flow and a fat phase, wherein the fat phase comprises a fat A and a fat B, wherein fat A comprises from 5 to 35% by weight pinolenic acid or a derivative thereof and wherein fat B has an N₂₀ value greater than 10.

## Description

This invention relates to a dough composition and to a process for its production

The nutritional value of the diet has come under increasing scrutiny. Food supplements are often taken by individuals in order to obtain nutritonal benefits. However, food supplements are typically in the form of capsules or the like and have the disadvantage that they are inconvenient in that an individual has to remember to take them. Food supplements of this type are typically not flavoured and are not attractive to many consumers.

Nutritional supplements have been incorporated into food products but the resulting food products can have an undesirable taste and the incorporation of the supplement can have a deleterious effect on the stability of the products.

Pinolenic acid (i.e., 5, 9, 12 C18:3 fatty acid, a fatty acid with 18 carbon atoms having three *cis* double bonds in the positions 5, 9 and 12) is present in, for example, pine nut oil and fractions thereof (see J Am Oil. Chem Soc 1998, 75, p.45-50). Pinolenic acid, as a highly unsaturated fatty acid, can be expected to suffer from the problem of low oxidative stability, Particularly when incorporated into foods and beverages.

In particular it would be expected that unsaturated fatty acids would suffer from low oxidative stability when exposed to conditions involving high temperatures, such as those used in the preparation of baked goods, such as breads and cookies.

FR-A-2756465 discloses the use of a concentrate with 15% pinolenic acid in various compositions, including food additives. The presence of pinolenic acid is described as providing a hypolipernic effect to the composition. There is no indication in the document as to how a food composition can be prepared and no examples are given.

EP-A-1685834 relates to the use of pinolenic acid and its derivatives for weight management by reducing the feeling of hunger and/or increasing satiety. A variety of product forms are mentioned.

JP 20031 16449 and JP10127227 disclose the use of fish oil in the preparation of bread.

EP 0 572 051 discloses a liquid improve comprising 75 to 95% by weight vegetable oil having an N₂₀ value of less than 2.0 (the N₂₀ value being the solid fat content as measured by NMR at 20°C).

It has now been found that a convenient vehicle for the consumption of pinolenic acid and its derivatives can be provided by certain dough compositions. Surprisingly, it is possible to incorporate the pinolenic acid or derivative in these compositions in relatively high amounts and yet still achieve good oxidative stability compared to other, less unsaturated oils. In other words, the pinolenic acid or derivative thereof has the same or better oxidative stability than oils with a similar level of unsaturation. The compositions also have good organoleptic properties (including taste and texture) and good stability.

Accordingly to one aspect of the present invention, there is provided a dough composition comprising flour and a fat phase, wherein the fat phase comprises from 5 to 35% by weight pinolenic: acid or a derivative thereof.

Conveniently, the dough composition further comprises water.

Advantageously, the dough composition comprises flour prepared from whole grain.

Preferably, the dough comprises brown flour.

Conveniently, the dough comprises white flour.

Preferably, the dough composition comprises a leavening agent.

Conveniently the leavening agent is yeast.

Advantageously, the flour is a mixture of wheat with other grains.

Preferably, the dough composition is free of wheat or gluten.

Conveniently, the dough composition is sourdough.

Advantageously, the dough composition comprises one or more additives selected from flavours, colouring agents, vitamins, acidity regulators, preservatives, emulsifiers, antioxidants, dietary fibres and mixtures thereof.

Preferably, the pinolenic acid is incorporated in the dough composition in the form of powder.

Conveniently, the dough composition further comprises a bread improver.

According to another aspect of the invention, there is provided a bread prepared using the dough composition of the invention.

According to a further aspect of the invention there is provided a bread improver comprising at least 8% pinolenic acid.

Preferably, the bread improver comprises ascorbic acid, sodium metabisulphate, ammonium chloride, amylase and/or protease.

According to another aspect of the invention, there is provided process for producing a dough composition of the invention which comprises admixing flour and a fat phase comprising from 5 to 35% by weight pinolenic acid or a derivative thereof to form the dough composition.

Preferably, the process further comprises baking the dough composition.

Conveniently, the pinolenic acid or derivative thereof is incorporated into the dough composition in the form of a powder.

Advantageously, the powder is produced by spray drying pinolenic acid or its derivatives, or a fat comprising pinolenic acid or its derivatives, with protein and/or carbohydrate.

According to another aspect of the invention, there is provided the use of a dough composition, a bread or a bread improver of the invention for a nutritional benefit.

Preferably, the benefit is a weight management effect.

According to a yet further aspect of the invention, there is provided a dough composition comprising flour and a fat phase, wherein the fat phase comprises a fat A and a fat B, wherein fat A comprises from 5 to 35% by weight pinolenic acid or a derivative thereof and wherein fat B has an N₂₀ value greater than about 10

Preferably, fat B has an N₂₀-value greater than about 20.

Conveniently, fat B has an N₂₀-value in the range of from about 30 to about 80.

Advantageously, the fat phase comprises a blend of fat A and fat B.

the fat phase comprises from 20 to 90% by weight fat B.

Conveniently, the fat phase comprises from 35 to 90% by weight fat B.

Advantageously, the fat phase comprises from 50 to 90% by weight fat B.

Preferably, fat B is selected from the group consisting of butter, cocoa butter equivalents, cocoa butter, palm oil or fractions thereof, palm kernel oil or fractions thereof, interesterified mixtures of above fats or fractions or hardened components thereof, and from liquid oils such as sunflower oil, high oleic sunflower oil, olive oil, soybean oil, rapeseed oil, cotton seed oil, safflower oil, high oleic safflower oil, maize oil and/or MCT oils and mixtures thereof.

Advantageously, the dough wherein the dough composition comprises flour that is prepared from whole grain.

Preferably, the dough composition comprises brown flour.

Conveniently, the dough composition comprises white flour.

Advantageously, the flour is a mixture of wheat with other grains.

Preferably, the dough composition is free of wheat or gluten.

Conveniently, the dough composition is sourdough.

Advantageously, the dough composition comprises one or more additives selected from flavours, colouring agents, vitamins, acidity regulators, preservatives, emulsifiers, antioxidants, dietary fibres and mixtures thereof.

Preferably, the pinolenic acid is incorporated in the dough composition in the form of a powder.

According to another aspect of the invention, there is provided a biscuit prepared using the dough composition of the invention.

According to a further aspect of the invention, there is provided a cake prepared using the dough composition of the invention.

According to yet another aspect of the invention there is provided puff pastry prepared using the dough composition of the invention.

According to a further aspect of the invention, there is provided a process for producing a dough composition of the invention which comprises admixing flout and a fat phase comprising a fat A and a fat B, wherein fat A comprises from 5 to 35% by weight pinolenic acid or a derivative thereof and wherein fat B has an N₂₀ value greater than about 15, to form the dough composition.

Preferably, the process further comprises baking the dough composition.

Conveniently, the pinolenic acid or derivative thereof is incorporated into the dough composition in the form of a powder.

Advantageously, the powder is produced by spray drying pinolenic acid or its derivatives, or a fat comprising pinolenic acid or its derivatives, with protein and/or carbohydrate.

Preferably, the process further comprises the step of mixing together fat A with fat B to give the fat phase before the fat phase is admixed with the flour.

Conveniently, the process comprises the steps of adding fat A and fat B to the flour separately.

According to another aspect of the invention, there is provided a use of a dough composition, a biscuit, a cake or puff pastry of the invention for a nutritional benefit.

Preferably, the benefit is a weight management effect.

Moreover, despite the level of unsaturation of pinolenic acid, the doughs have unexpectedly good oxidative stability when compared to other oils, such as high oleic sunflower oil, sunflower oil, CLA and fish oils.

A preferred dough composition of the invention is bread dough, which may subsequently be baked to give bread. The dough composition of the invention comprises flour and a fat phase. The dough composition may be used to form breads by baking steaming, frying or otherwise cooking the dough. The dough may comprise a leavening agent to allow the dough to rise, or a leavening agent may be absent, for example in the case of the preparation of a flat bread. The dough compositions may comprise flour comprising one or more type of grain. Preferred grains include wheat, rye, barley, maize, spelt and maize, and mixtures thereof. The flours may be whole grain, i.e. made from the entire grain including bran, endosperm and germ, or may be processed to partially or substantially remove the bran and/or germ to produce processed flours, including brown flour and white flour.

The dough compositions of the invention may be used to produce a variety of breads, including flat breads such as naan, roti, poppadum, pita, chappati, foccacia, poori, pizza and tortilla. The bread may have been made from a risen dough such as a dough made using yeast or a sour dough, such as ciabatta, french bread, such as baguettes, bagels, brioche, pizza, pretzel, and can be in forms such as batch loaves, rolls and sticks.

Dough compositions of the invention optionally comprise one or more additional additives selected from flavours, colouring agents, vitamins, minerals, acidity regulators, preservatives, emulsifiers, antioxidants, dietary fibres and mixtures thereof. Each of these materials may be a single component or a mixture of two or more components.

Examples of suitable vitamins and minerals include calcium, iron, zinc, copper, phosphorous, biotin, folic acid, pantothenic acid, iodine, vitamin A, vitamin C, vitamin B1, vitamin B2, vitamin B3, vitamin B6, vitamin B9, vitamin B12, Vitamin D, vitamin E, and vitamin K. Preferably, when a vitamin or mineral is utilized the vitamin or mineral is selected from iron, zinc, folic acid, iodine, vitamin A, vitamin C, vitamin B2, vitamin B3, vitamin B6, vitamin B12, vitamin D, and vitamin E.

Acidity regulators include organic as well as inorganic edible acids. The acids can be added or be present in their undissociated form or, alternatively, as their respective salts, for example, potassium or sodium hydrogen phosphate, potassium or sodium dihydrogen phosphate salts. The preferred acids are edible organic acids which include citric acid, malic acid, fumaric acid, adipic acid, phosphoric acid, gluconic acid, tartaric acid, ascorbic acid, acetic acid, phosphoric acid, or mixtures thereof. Glucono Delta Lactone (GDL) may also be used, particularly wherein it is desired to reduce pH without introducing excessive acidic, or tart, flavour in the final composition.

Colouring agents including natural and artificial colours may optionally be used. Non-limiting examples of colouring agents include fruit and vegetable juices, riboflavin, carotenoids (e.g. p-carotene), tumeric, and lycopenes.

Dietary fibres are complex carbohydrates resistant to digestion by mammalian enzymes, such as the carbohydrates found in plant cell walls and seaweed, and those produced by microbial fermentation.

Preservatives may be selected from the group consisting of sorbate preservatives, benzoate preservatives, and mixtures thereof.

Antioxidants include, for example, natural or synthetic tocopherols, TBHQ, BUT, BHA, free radical scavengers, propylgallate, ascorbylesters of fatty acids and enzymes with anti-oxidant properties.

As used herein, the term liquid oil' refers to a fat that is liquid at room temperature, i.e. at about 25°C. Examples of liquid oils include vegetable oils such as extra virgin olive oil, virgin olive oil, native olive oil and peanut oil.

Pinolenic acid or a derivative of pinolenic acid is an essential component of the compositions of the invention. The pinolenic acid or derivative (which term is intended to cover both pinolenic acid and derivatives when both are preset) is preferably in a form selected from the free acid, salts, mono-, di- or triglycerides, or mixtures thereof.

Sources of pinolenic acid and its derivatives are available and will be known to those skilled in the art. Preferably, the pinolenic acid or derivative is in the form of pine nut oil or is derived from pine nut oil.

The pinolenic acid or derivative in the dough compositions may form part of a fat composition that comprises one or more other components,

The physical properties of fats can be defined in terms of an N value. These values indicate the percentage of solid fat present in a composition at a certain temperature. Thus, the term Nₓ refers to solid fat content at a temperature of x°C, measured by NMR pulse techniques on unstabilised fats. In this context, an 'unstabilised fat' refers to a fat that was melted at 80°C, kept at 60°C for 5 minutes, cooled to 0°C, kept at 0°C for 1 hour, and then kept at the measurement temperature x°C for 30 minutes before NMR analysis.

The pinolenic acid or derivative is in the form of a fat which comprises from 5 to 35 %, more preferably from 10 to 30%, such as from 13 to 25 %, or from 13 to 20%, by weight pinolenic acid or derivative thereof, based on the total weight of fatty acids in the fat (calculated using fatty acid methyl ester analysis).

The preferred form of pinolenic acid for use in the invention is as a glyceride. Particularly preferred are diglycerides and triglycerides, with triglycerides being even more preferred.

Examples of other fatty acids that may be present in the fat include linoleic acid, oleic acid, taxoleic, juniperonic, sciadonic, saturated fatty acids, conjugated linoleic acid (optionally as an enriched isomer mixture) and EPA (eicosapentaenoic) and DHA (docosahexaenoic).

Particularly preferred fats used in dough compositions of the invention are those in which the pinolenic acid or derivative is in the form of a composition which additionally comprises from 30 to 70 % by weight linoleic acid or derivative thereof, based on the total weight of fatty acids in the fat (calculated as fatty acid methyl esters). Additionally or alternatively, the pinolenic acid or derivative is in the form of a fat which additionally comprises from 10 to 40 % by weight oleic acid or derivative thereof, based on the total weight of fatty acids in the fat (calculated as free fatty acid). Additionally or alternatively, the pinolenic acid or derivative is in the form of a fat which additionally comprises from 1 to 15 % by weight palmitic acid or derivative thereof, based on the total weight of fatty acids in the fat (calculated using fatty acid methyl ester analysis). Additionally or alternatively, the fat may comprise from 0.5 to 5 wt% of taxoleic acid or a derivative thereof.

Specific examples of fats comprising pinolenic acid or a derivative thereof that are useful in the invention include the following:
- Fat compositions comprising from 5 to 35 %, more preferably from 15 to 30 %, by weight pinolenic acid or a derivative, thereof, together with from 30 to 70 % by weight linoleic acid or a derivative thereof;
- Fat compositions comprising from 5 to 35 %, more preferably from 15 to 30%, by weight pinolenic acid or a derivative thereof, together with from 10 to 40 % by weight oleic acid or a derivative thereof;
- Fat compositions comprising from 5 to 35 %, more preferably from 15 to 30 %, by weight pinolenic acid or a derivative thereof, together with from 1 to 15 % by weight palmitic acid or a derivative thereof;
- Fat compositions comprising from 5 to 35 %, more preferably from 15 to 30 %, by weight pinolenic acid or a derivative thereof, together with from 0.5 to 5 wt% of taxoleic acid or a derivative thereof;
- Fat compositions comprising from 5 to 35 %, more preferably from 15 to 30 %, by weight pinolenic acid or a derivative thereof, together with from 30 to 70 % by weight linoleic acid or a derivative thereof and from 10 to 40 % by weight oleic acid or a derivative thereof,
- Fat compositions comprising from 5 to 35 %, more preferably from 15 to 30 %, by weight pinolenic acid or a derivative thereof together with from 3 0 to 70 % by weight linoleic acid or a derivative thereof and from 1 to 15 % by weight palmitic acid or a derivative thereof;
- Fat compositions comprising from 5 to 35 %, more preferably from 15 to 30 %, by weight pinolenic acid or a derivative thereof together with from 34 to 70 % by weight linoleic acid or a derivative thereof and from 0.5 to 5 wt% of taxoleic acid or a derivative thereof;
- Fat compositions comprising from 5 to 35 %, more preferably from 15 to 30 %, by weight pinolenic acid or a derivative thereof together with from 30 to 70 % by weight linoleic acid or a derivative thereof, from 1 to 15 % by weight palmitic acid or a derivative thereof and from 10 to 40 % by weight oleic acid or a derivative thereof; and
- Far compositions comprising from 5 to 35 %, more preferably from 15 to 30 %, by weight pinolenic acid or a derivative thereof, together with from 30 to 70 % by weight linoleic acid or a derivative thereof, from 1 to 15 % by weight palmitic acid or a derivative thereof, from 10 to 40 % by weight oleic acid or a derivative thereof and from 0.5 to 5 wt% of taxoleic acid or a derivative thereof.

In these fats, the amounts of the acids or derivatives are determined as fatty acid methyl esters based on the total fatty acid and/or derivative content of the fat. Preferably, the fatty acids are present as glycerides (more preferably triglycerides) (i.e., more than 90 %, preferably more than 95 %, by weight of the fatty acids are present as glycerides, more preferably triglycerides). Another preferred glyceride is the diglyceride.

In a preferred embodiment of the invention, the pinolenic acid or derivative represents at least 75 % by weight of the total Δ5-polyunsaturated C18-C20 fatty acids in the fat (calculated as fatty acid methyl esters).

The pinolenic acid used in the present invention may be in the form of a free fatty acid, a derivative of panolenic acid or mixtures thereof, including mixtures of different derivatives. Derivatives are non-toxic and edible. Derivatives of pinolenic acid, which can be used in the present invention, include salts of pinolenic acid and esters. Suitable salts include salts with food grade cations such as sodium salts and calcium salts. Suitable esters include alkyl esters having from one to six carbon atoms. Preferred derivatives are esters and preferred esters are mono-, di- and tri- glycerides and mixtures thereof.

The other fatty acid or each of the other fatty acids in the fat can independently be present as a free fatty acid or as a derivative thereof (including a mono-, di- or triglyceride and salts, preferably glycerides), or as a mixture thereof.

A suitable source for the pinolenic acid used in the present invention is pine nut oil or concentrates thereof. For example, glycerides of pinolenic acid can be obtained from pine nut oil or concentrates thereof. Preferably, an oil or concentrate with a content of pinolenic acid or a derivative thereof of more than 15 % by weight or more than 28 % by weight is used (such as up to 50 % by weight).

Concentrates of pinolenic acid or a derivative thereof that may be used in the present invention can be prepared by any suitable process. A suitable process is described in EP-A-1088552.

In one suitable process, an enzymic hydrolysis or glycerolysis is performed using an enzyme that can discriminate between fatty acids with a delta 5 double bond and other fatty acids. This process comprises:
i) reacting a glyceride material containing at least 2 % by weight of fatty acid with cis⁵ double bond with water or glycerol in the presence of an enzyme capable of discriminating between fatty acids containing a delta 5 double bond and other fatty acids;
ii) splitting the reaction mixture into a partial glyceride rich component and a fatty acid rich component;
iii) optionally converting the partial glycerides of step ii) to free fatty acids in the presence of a suitable enzyme;
iv) optionally converting the fatty acid rich component of step ii) to triglycerides by reaction with glycerol in the presence of a suitable catalyst such as a suitable enzyme; and
v) optionally splitting the partial glyceride rich material of step ii) into components that are a) rich in monoglycerides, b) rich in
diglycerides and c) rich in triglycerides and then optionally converting the partial glycerides a) and b) into triglycerides by reaction with fatty acids in the presence of a suitable enzyme.

It is preferred to use a glyceride material with a pinolenic acid content of 5 to 50 wt %, preferably 10 to 35 wt % in step i). Examples of such materials are pinolenic oils and concentrates thereof. This process produces a concentrate that contains at least 28 % by weight pinolenic acid.

Enzymes suitable for use in steps i), iii), iv) and v) are lipases. Suitable commercial lipases include *Candida rugosa* lipase; Lipase QL; Lipase SL, Lipase OF; *Rhizopus delemar*; lipase; *Rhizopus oryzae* lipase; *Geotrichum candidum* B lipase; and *Rhizomucor miehei* lipase. Preferred enzymes for step i) are *Candida rugosa* lipase and *Geotrichum candidum* B lipase.

Suitable lipases also include Lipozyme IM (a commercial enzyme). The preferred enzyme for use in step iv) is Lipozyme M (from *Rhizomucor miehei*).

The fats comprising pinolenic acid or a derivative thereof that are useful in the invention may comprise one or more otter fatty acids. The term fatty acid, as used herein, refers to straight chain carboxylic acids having from 12 to 24 carbon atoms and being saturated or unsaturated e.g., having 0, 1, 2 or 3 double bonds.

The pinolenic acid or derivative thereof is optionally blended with additional fatty acids or glycerides before being used in the fat of the present invention. When the compositions contain one or more fatty acids and/or glycerides in addition to the pinolenic acid or derivative thereof the additional fatty acid(s) and/or glycerides are preferably selected from liquid oils, such as soybean oil, sunflower oil, rape seed oil, olive oil, flax seed oil and cotton seed oil; cocoa butter and cocoa butter equivalents; palm oil and fractions thereof, enzymically made fats; fish oils and fractions thereof; conjugated linoleic acid and enriched isomer mixtures; gamma linolenic acid and enriched mixtures thereof; hardened liquid oils; and mixtures thereof.

The pinolenic acid or derivative thereof can be included in the dough of the invention as oil or in the form of a powder, such as a free flowing powder. Pinolenic acid and its derivatives in powder form can be produced, for example, by spray drying pinolenic acid or its derivatives, or a fat comprising pinolenic and or its derivatives, with protein and/or carbohydrate, with the powder typically comprising from 50 to 90% by weight of fat. It has been found that use of the powder can give extra stability to the dough.

The dough composition of the invention can be used to prepare products having advantageous properties; the dough can be cooked in a known manner such as baking and frying to give various cooked goods such as bread, biscuits (also referred to as cookies), cakes and puff pastry. The cooked goods have improved characteristics, such as softness, crispness and crunchiness. The cooked goods may also slice well when cut with a knife. The goods may also have good organoleptic characteristics, such as easily dissolving in the mouth without being too dry or damp, and breaking easily (snap). Other advantageous properties include good volume, brittleness, firmness, crispiness and compacity.

The invention may have the beneficial property of preventing the dough and the cooked goods from drying too quickly, The cooked goods also have good crust properties without being too crumbly or crispy.

The use of pinolenic acid or a derivative thereof also avoids problems of stability encountered with other fats, such as fish oil. The dough compositions of the invention, and goods prepared therefrom exhibit good flavour and taste characteristics.

Also, the dough compositions themselves have improved characteristics, such as in terms of consistency, elasticity and aeration properties.

The listing or discussion of an apparently prior-published document in this specification should not necessarily be taken as an acknowledgement that the document is part of the state of the art or is common general knowledge.

The following non-limiting examples illustrate the invention and do not limit its scope in any way. In the examples and throughout this specification, all percentages, parts and ratios are by weight unless indicated otherwise.

### Example

### Example 1

### Bread example with PinnoThin

### WHOLE GRAIN BREAD WITH PINNOTHIN^{™}

| **% by weight** | **Ingredient** |
|---|---|
| 37 | Wholemeal flour |
| 16 | Multi-grain mix |
| 3.1 | PinnoThin™ |
| 2.2 | Gluten powder |
| 1.6 | Bread improver |
| 1.6 | Yeast |
| 1.1 | Salt |
| 37.4 | Water till 100% |

The multi-grain mix is soaked in water (27% on flour) for at least 30 minutes. Then all the ingredients are mixed with water (36% on flour), spiral kneaded 3 minutes slow and 3 minutes fast. The rest of water is added (7% on flour) and mixed 1 minute slow and 5 minutes fast. The dough is scaled, formed and leavened for 45 minutes. The dough is formed to long shape and put in baking tin. The load is leavened for another 70 minutes. The baking takes place at 250/220°C for 30 minutes.

### Example 2

### Wholemeal cookies with PinnoThin™

| **% by weight** | **Ingredient** |
|---|---|
| 38.8 | Whole wheat flour |
| 20.0 | Flour |
| 20.0 | Butter |
| 17.7 | Eggs |
| 3.5 | PinnoThin™ (pine nut oil) |

PinnoThin™ is weighed and mixed with the flour. All other ingredients are added and mixed in mixer to form a sweet short pastry. Then the pastry is cooled to 4°C / 39°F. The pastry is rolled out to a thickness of 5-6 mm. Pieces are cut out and brushed with egg yolk. The cookies are baked at 180°C / 356°F for 15 minute (fan oven). The wholemeal cookies are packed in polypropylene bags.

PinnoThin^{™} , a mixture of triglyderides, is a trademark of Lipid Nutrition BV and has the following fatty acid composition measured as fatty acid methyl ester (in weight %):

| | |
|---|---|
| Pinolenic acid | 16 |
| Linoleic acid | 46 |
| Oleic acid | 25 |
| Palmitic acid | 4 |
| Taxoleic acid | 2 |
| Others | Balance to 100 |

## Claims

1. A dough composition comprising flour and a fat phase, wherein the fat phase comprises from 5 to 35% by weight pinolenic acid or a derivative thereof.

2. A dough composition as claimed in Claim 1 which further comprise water.

3. A dough composition as claimed in any of the preceding claims, wherein the dough composition comprises flour prepared from whole grain.

4. A dough composition as claimed in any of the preceding claims, wherein the dough comprises brown flour.

5. A dough composition as claimed in any of the preceding claims, wherein the dough comprises white flour.

6. A dough composition as claimed in any of the preceding claims, which comprises a leavening agent.

7. A dough composition as claimed in Claim 6, wherein the leavening agent is yeast.

8. A dough composition as claimed in any of the preceding claims, wherein the flour is a mixture of wheat with other grains.

9. A dough composition as claimed in any of Claims 1 to 7, which is free of wheat or gluten.

10. A dough composition as claimed in any of the preceding claims, which is sourdough.

11. A dough composition as claimed in any of the preceding claims, comprising one or more additives selected from flavours, colouring agents, vitamins, acidity regulators, preservatives, emulsifiers, antioxidants, dietary fibres and mixtures thereof.

12. A dough composition as claimed in any of the preceding claims, wherein the pinolenic acid is incorporated in the dough composition in the form of a powder.

13. A dough composition as claimed in any of the preceding claims further comprising a bread improver.

14. A bread prepared using the dough composition according to any of the preceding claims.

15. A bread improver comprising at least 8% pinolenic acid.

16. A bread improver as claimed in Claim 15 which comprises ascorbic acid, sodium metabisulphate, ammonium chloride, amylase and/or protease.

17. A process for producing a dough composition as defined in any of Claims 1 to 13, which comprises admixing flour and a fat phase comprising from 5 to 35% by weight pinolenic acid or a derivative thereof to form the dough composition.

18. A process as claimed in Claim 17, further comprising baking the dough composition.

19. A process as claimed in Claim 17 or 18, wherein the pinolenic acid or derivative thereof is incorporated into the dough composition in the form of a powder.

20. A process as claimed in Claim 19, wherein the powder is produced by spray drying pinolenic acid or its derivatives, or a fat comprising pinolenic acid or its derivatives, with protein and/or carbohydrate.

21. Use of a dough composition any of Claims 1 to 13 or a bread according to Claim 14, or a bread improver according to Claim 15 or 16.

22. Use as claimed in Claim 21, wherein the benefit is a weight management effect.

23. A dough composition comprising flour and a fat phase, wherein the fat phase comprises a fat A and a fat B, wherein fat A comprises from 5 to 35% by weight pinolenic acid or a derivative thereof and wherein fat B has an N₂₀ value greater than about 10.

24. A dough composition according to Claim 23, wherein fat B has an N₂₀-value greater than about 20.

25. A dough composition according to Claim 23 or 24, wherein fat B has an N₂₀-value in the range of from about 30 to about 80.

26. A dough composition according to any of Claims 23 to 25, wherein the fat phase comprises a blend of fat A and fat B.

27. A dough composition as claimed in any of Claims 23 to 26 wherein the fat phase comprises from 20 to 90% by weight fat B.

28. A dough composition as claimed in any of Claims 23 to 27 wherein the fat phase comprises from 35 to 90% by weight fat B.

29. A dough composition as claimed in any of Claims 23 to 28 wherein the fat phase comprises from 50 to 90% by weight fat B.

30. A dough composition as claimed in any of Claims 23 to 29, wherein fat B is selected from the group consisting of butter, cocoa butter equivalents, cocoa butter, palm oil or fractions thereof, palm kernel oil or fractions thereof, interesterified mixtures of above fats or fractions or hardened components thereof, and from liquid oils such as sunflower oil, high oleic sunflower oil, olive oil, soybean oil, rapeseed oil, cotton seed oil, safflower oil, high oleic safflower oil, maize oil and/or MCT oils and mixtures thereof.

31. A dough composition as claimed in any of Claims 23 to 30, wherein the dough composition comprises flour that is prepared from whole grain.

32. A dough composition as claimed in any of Claims 23 to 31 wherein the dough composition comprises brown flour.

33. A dough composition as claimed in any of Claims 23 to 32 wherein the dough composition comprises white flour.

34. A dough composition as claimed in any of Claims 23 to 33, wherein the flour is a mixture of wheat with other grains.

35. A dough composition according to any of Claims 23 to 34, which is free of wheat or gluten.

36. A dough composition according to any of Claims 23 to 35, which is sourdough.

37. A dough composition as claimed in any of Claims 23 to 36, comprising one or more additives selected from flavours, colouring agents, vitamins, acidity regulators, preservatives, emulsifiers, antioxidants, dietary fibres and mixtures thereof.

38. A dough composition as claimed in any of Claims 23 to 37, wherein the pinolenic acid is incorporated in the dough-composition in the form of a powder.

39. A biscuit prepared using the dough composition as claimed in any of Claims 23 to 38.

40. A cake prepared using the dough composition as claimed in any of Claims 23 to 38.

41. Puff pastry prepared using the dough composition as claimed in any of Claims 23 to 38.

42. A process for producing a dough composition as defined in any of Claims 23 to 38, which comprises admixing flour and a fat phase comprising a fat A and a fat B, wherein fat A comprises from 5 to 35% by weight pinolenic acid or a derivative thereof and wherein fat B has an N₂₀ value greater than about 15, to form the dough composition.

43. A process as claimed in Claim 42, further comprising baking the dough composition.

44. A process as claimed in any of Claims 42 or 43, wherein the pinolenic acid or derivative thereof is incorporated into the dough composition in the form of a powder.

45. A process as claimed in Claim 44, wherein the powder is produced by spray drying pinolenic acid or its derivatives, or a fat comprising pinolenic acid or its derivatives, with protein and/or carbohydrate.

46. A process according to any of Claims 42 to 45, which further comprises the step of mixing together fat A with fat B to give the fat phase before the fat phase is admixed with the flour.

47. A process according to any of Claims 42 to 45, which comprises the steps of adding fat A and fat B to the flour separately.

48. Use of a dough composition according to any of Claims 25 to 38, a biscuit as claimed in Claim 39, a cake as claimed in Claim 40 or puff pastry as claimed in Claim 41 for a nutritional benefit.

49. Use as claimed in Claim 48, wherein the benefit is a weight management effect.
